# EUROPEAN PATENT SPECIFICATION

(11) **EP 3 181 524 B1**
(45) Date of publication and mention of the grant of the patent: **24.04.2019**
(21) Application number: 15200000.6
(22) Date of filing: 15.12.2015
(51) Int. Cl.: C02F 3/28, A01C 3/00, B09B 3/00, C05F 3/00, C05F 17/00, C02F 11/04, C12M 1/107, C12M 1/00, C12M 1/34, C12P 5/02, C02F 103/20

(54) **METHOD FOR TREATMENT UNDER PRESSURE OF LIQUID FEEDSTOCK**
VERFAHREN ZUR BEHANDLUNG DES FLÜSSIGEN ROHSTOFFES UNTER DRUCK
PROCEDE POUR LE TRAITEMENT SOUS PRESSION DE CHARGE LIQUIDE

(43) Date of publication of application: 21.06.2017
(73) Proprietor: Holzer, André, Saint Petersburg 195027 (RU); Kohleick, Reinhard, 42109 Wuppertal (DE); Millett, Thomas, 1469 Luxembourg (LU)
(72) Inventor: Holzer, André, Saint Petersburg 195027 (RU); Kohleick, Reinhard, 42109 Wuppertal (DE); Millett, Thomas, 1469 Luxembourg (LU)
(74) Representative: Mellet, Valérie Martine

(56) References cited:
- AU-A1- 2014 200 009
- US-A1- 2002 079 266
- V.A. VAVILIN ET AL: "Modelling of gas pressure effects on anaerobic digestion", BIORESOURCE TECHNOLOGY., vol. 52, no. 1, 1 January 1995 (1995-01-01), pages 25-32, XP055247706, GB ISSN: 0960-8524, DOI: 10.1016/0960-8524(94)00148-T
- John P Chastain ET AL: "Poultry Manure Production and Nutrient Content", , 17 December 2004 (2004-12-17), pages 1-17, XP055247705, Retrieved from the Internet: URL:https://www.clemson.edu/extension/live stock/camm/camm_files/poultry/pch3b_00.pdf [retrieved on 2016-02-04]

## Description

### Field of the invention

The present invention relates to a process for the treatment of organic waste with a fermentation gas fermenter (or biogas reactor). The invention is particularly suitable, but not only, for the production of methane via biomethanation processes and plants.

### Background of the invention

Biomethanation is an effective and ecological method for the treatment of organic wastes while producing energy in the form of methane via methanogenic bacteria. The methanation allows treating refuses and wastes as diverse as wastes materials, sewage sludge, animal wastes, food processing industries wastes, household wastes, agricultural wastes, etc.

Liquid wastes can be treated by the methanation even when they show suspended material or dry matter, as animal wastes (e.g. liquid manure) or sewage sludge. In order to have the best fermentation conditions, it is common practice to mix wastes from different origin. For example, agricultural wastes can be mixed with animal wastes which contain fat showing a high methanogenic potential. Since the wastes are liquid or treated to be liquid, a good homogeneity can be achieved by agitating. The mixing operation typically occurs in a pre-treatment tank. The mixed liquid waste is then pumped to be directed to a biogas reactor where it is subjected to anaerobic fermentation.

One of the problems encountered in the prior art is the treatment of nitrogen-containing biomass such as chicken manure, because ammonium is known to inhibit the methanogenic bacteria even at low concentration.

Chicken manure has the highest amount of nitrogen, phosphorus and potassium out of all kinds of animal manure. In *"*Available nitrogen from animal manures" from Parker F. Pratt and Javier Z. Castellanos in CALIFORNIA AGRICULTURE, JULY-AUGUST 1981 the following content of total nitrogen is disclosed: 4,6 % on dry weight for chicken manure; 2.6 % on dry weight for beef manure.

This is why it is common practice to mix chicken manure with other agricultural wastes for biogas production. A process wherein chicken manure is mixed with cotton burrs is described in US6299774 for instance. It can be seen from the examples that in such a process the content of agricultural waste is predominant as about 6.5 kg (14.5 lbs) of chicken manure is mixed with 90.5 kg (200 lbs) of agricultural waste. Thus there is a need for a process allowing the treatment of high nitrogen-containing biomass per se, i.e. without the need of diluting it in lower nitrogen-containing wastes.

DE102012211781 suggests to submit the biomass to both ammonia fermentation and biogas fermentation subsquently. During the ammonia fermentation step, ammonia is removed by means of stripping gas and/or by precipitation from the biomass in order to have the biomass's nitrogen content depleted. Then, in a subsequent step, the biomass is subjected to a biogas fermentation.

EP2666868 discloses the fermentation of nitrogenous biomass in a biogas plant. The system comprises mixing means adapted to keep the nitrogen concentration below a defined threshold before the biomass is fed to the biogas plant.

In WO2012152266, a fermentation substrate is mixed with reduced-ammonia fermentation liquid supplied in at least an amount via which a loading of 2.5 g of NH₄ per liter or below is set in the fermentation substrate. The resultant fermentation substrate is subjected to a first fermentation during a time period from one to four days at a pH of below 6.5, wherein a hydrolysis and acidogenesis take place and a weak gas is produced as first biogas stream. The fermented fermentation substrate is then pumped into a following fermenter and in this is subjected to a further fermentation, wherein further acidogenesis, acetogenesis and methanogenesis take place. In this process a second biogas stream, having a lower hydrogen sulphide content than the weak gas, is generated.

Ammonia inhibition has been studied in connection with the digestion of chicken Manure, such as in *"*Effect of Temperature and Carbon-Nitrogen (C/N) Ratio on the Performance of Anaerobic Co-Digestion of Dairy Manure, Chicken Manure and Rice Straw: Focusing on Ammonia Inhibition" Xiaojiao Wang and al. PLOS ONE May 2014, Vol. 9, Issue 5, e97265. This study suggests firstly an effect of the temperature on the biogas production i.e. that increased temperature from mesophilic to thermophilic conditions resulted in ammonia inhibition. Secondly, the study suggests that this kind of inhibition could be reduced or avoided by increasing the C/N ratio of the mixed feedstock to an appropriate level. The adjustment of the C/N ratio was done by mixing in appropriate proportions of chicken manure showing a C/N of 9.6 with dairy manure showing a C/N of 22.2 and rice straw showing a C/N of 51.7. According to the results of this study ammonia inhibition occurred with a C/N ratio of 20 at 55°C, whereas a C/N ratio of 15 experienced inhibition at 35ºC.

US2002/0079266 and AU 2014 200009 are further examples of relevant prior art concerned with anaerobic digestion.

Thus, at the current state of the art, the content of the nitrogen-containing biomass that can be used in a biogas reactor is limited by a threshold beyond which fermentation is inhibited. So, there is still a need to find a solution in order to overcome ammonia inhibition limit in fermentation.

### Summary of the invention

According to a first aspect the invention provides a process for treating a feedstock in a liquid form comprising the steps of:
- providing a feedstock;
- optionally diluting the feedstock by addition of an aqueous solution in order to obtain a feedstock slurry, and
- subjecting the feedstock or the feedstock slurry to an anaerobic fermentation in a biogas reactor thereby forming a liquid phase and gas phase,
wherein the biogas reactor is supplied with feedstock or feedstock slurry in at least an amount via which content of ammonium of at least 2.7 g/l as determined according to DIN 38406-5 is set in the liquid phase and wherein the anaerobic fermentation is conducted at a pressure of at least 0.13 MPa and of at most 1.00 MPa of the gas phase in the head space of the biogas reactor, wherein that the feedstock and/or the feedstock slurry (11) has a C/N ratio of at most 15 wherein C is the total carbon and is determined according to DIN ISO 10694 and N is the total nitrogen and is determined according to DIN ISO 13878, and wherein the feedstock is an organic waste mixture containing at least 60 wt% of poultry manure based on the total weight of the mixture

Surprisingly, it has been discovered that pressure fermentation, and preferably high-pressure fermentation, allows fermentation of high nitrogen-containing biomass such as poultry manure or chicken manure while keeping it predominant in the mixture used (i.e. at more than 50 wt% of the mixture) or even without the need of diluting it in lower nitrogen-containing wastes. Whereas prior art recommends to stay below a defined content of ammonium, it has been discovered that it is possible to conduct an anaerobic fermentation in a biogas reactor with a liquid phase having a content of ammonium above the threshold usually observed in prior art. In the invention, the feedstock and/or the feedstock slurry has a C/N ratio of at most 15. Whereas prior art studies demonstrated that ammonia inhibition could be avoided by increasing the C/N ratio of the material digested, the invention demonstrates an influence of pressure that allows the fermentation to be performed on slurry showing low C/N ratio. According to the invention, ammonia inhibition is avoided in both mesophilic and thermophilic conditions for slurry showing a C/N ratio of less than 15 and even of less than 10 under a pressure of at least 0.13 MPa, preferably at least 0.25 MPa. Additionally, the advantage of the invention is to provide a one step process which does not need any ammonia stripping or any acidification step to perform hydrolysis, acidogenosis and acetogenosis, before the biomass is subjected to fermentation.

Preferred embodiments of the process according to the invention are set herein below. Each embodiment of the invention so defined may be combined with any other embodiment, unless clearly indicated to the contrary.
- The feedstock and/or the feedstock slurry has a C/N ratio of at most 12, preferably of at most 10, wherein C is the total carbon and is determined according to DIN ISO 10694 and N is the total nitrogen and is determined according to DIN ISO 13878.
- The feedstock is animal manure, preferably poultry manure, more preferably chicken manure.
- The feedstock is an organic waste mixture containing at least 60 wt% of chicken manure, based on the total weight of the mixture.
- The aqueous solution is water such as fresh or recycled water.
- To obtain a feedstock slurry, the feedstock is diluted in water such as fresh or recycled water with at least 40 wt% of water, preferably with at least 50 wt% of water, based on the total weight of the feedstock slurry.
- The feedstock is diluted in water such as fresh or recycled water to obtain a feedstock slurry. The feedstock is diluted with at most 70 wt% of water, preferably with at most 60 wt% of water, based on the total weight of the feedstock slurry.
- The anaerobic fermentation is conducted with the anaerobic bacteria content of the feedstock and no additional anaerobic bacteria is inoculated in the biogas reactor, or the biogas reactor is inoculated with anaerobic bacteria, with preference with thermophile anaerobic bacteria.
- The process is a mesophilic process conducted at temperatures ranging from 20 to 40°C.
- The process is a thermophilic process conducted at temperatures ranging from 45 to 80°C, preferably from 50 to 70°C more preferably from 53 to 60°C.
- The anaerobic fermentation is conducted at a pH ranging from 6 to 8.5, preferably from 7 to 8.
- The pressure of the gas phase in the head space of the biogas reactor is of at least 0.14 MPa, preferably at least 0.15 MPa, preferably at least 0.20 MPa, preferably at least 0.25 MPa, preferably at least 0.28 MPa, preferably at least 0.30 MPa, more preferably at least 0.32 MPa, even more preferably of at least 0.35 MPa, and most preferably of at least 0.40 MPa.
- The pressure of the gas phase in the head space of the biogas reactor is of at most 1.00 MPa.
- The biogas reactor is supplied with feedstock or feedstock slurry in at least an amount via which content of ammonium of at least 2.8 g/l is set in the liquid phase, preferably of at least 3.0 g/l, more preferably of at least 3.5 g/l.
- The anaerobic fermentation is a one phase anaerobic digestion.

The process as defined above for treating a feedstock in a liquid form can be conducted in a plant comprising:
- optional means to provide feedstock and an aqueous solution to a collection tank provided with agitating means in order to form a feedstock slurry;
- at least one pressurizable biogas reactor comprising a liquid phase and a gas phase;
- a pressurizer associated to each pressurizable biogas reactor and means to monitor the pressure of the gas phase in said associated biogas reactor configured to act on the pressurizer in order to keep a pressure of at least 0.13 MPa, preferably at least 0.25 MPa of the gas phase in the head space of the biogas reactor;
wherein the plant further comprises a feedstock or feedstock slurry feeder in order to supply the biogas reactor with feedstock slurry, wherein the feedstock or feedstock slurry feeder is configured to supply said feedstock or feedstock slurry at least in an amount via which content of ammonium of at least 2.7 g/l as determined according to DIN 38406-5 is set in the liquid phase of the biogas reactor.

According to a preferred embodiment, the plant further comprises an ultrafiltration unit and a reverse osmosis unit to treat the digestate exiting the biogas reactor.

Optionally, the plant further comprises a centrifuge to treat the digestate before the ultrafiltration unit and means to redirect the centrifuge concentrate to the biogas reactor.

In an embodiment, the plant may comprises a self-cleaning filter before the ultrafiltration unit.

In a preferred embodiment the pressurizer is one or more selected from the group consisting of the feedstock or feedstock slurry feeder; an external compressed gas source or a fluid inlet.

In another preferred embodiment, the feedstock or feedstock slurry feeder comprises dosing means to mix one or more organic waste in selected amounts in order to form the feedstock and/or to dilute the feedstock with water thereby forming the feedstock slurry.

In another preferred embodiment, the means to provide the feedstock comprise mixing means to mix poultry manure, preferably chicken manure with one or more organic waste material wherein the mixing means are configured to provide a mixture containing at least 60 wt% of poultry manure, preferably chicken manure, based on the total weight of the mixture.

In another preferred embodiment the plant comprises only one biogas reactor.

### Brief description of the drawings

- Figure 1 represents a plant that can be use to perform the process according to the invention
- Figure 2 represents a graph showing the production in vol% of NH₄ and CO₂ in a biogas fermentation process according to the invention as a function of time, demonstrating the influence of the pressure on methanation.

### Detailed description of the invention

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" also include the term "consisting of".

The recitation of numerical ranges by endpoints includes all numbers and fractions subsumed within the respective ranges, as well as the recited endpoints.

The particular features, structures, characteristics or embodiments may be combined in any suitable manner, as would be apparent to a person skilled in the art from this disclosure, in one or more embodiments.

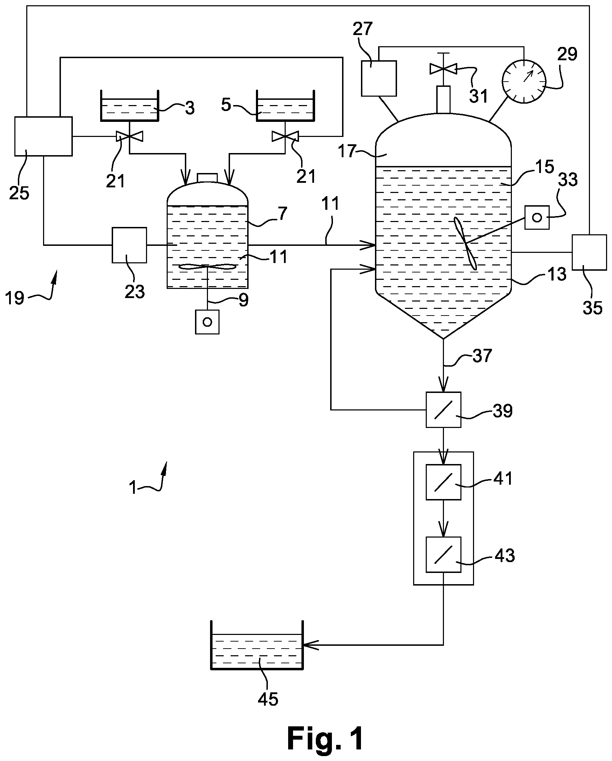

The plant 1 and the process according to the invention are described jointly with reference to figure 1.

The process according to the invention allows treating a feedstock in a liquid form and comprises the steps of:
- providing a feedstock;
- optionally diluting the feedstock by addition of water in order to obtain a feedstock slurry, and
- subjecting the feedstock or feedstock slurry to an anaerobic fermentation in a biogas reactor thereby forming a liquid phase and gas phase, wherein the biogas reactor is supplied with feedstock or feedstock slurry at least in an amount via which content of ammonium of at least 2.7 g/l as determined according to DIN 38406-5 is set in the liquid phase and the anaerobic fermentation is conducted at a pressure of at least 0.13 MPa, preferably at least 0.25 MPa, and of at most 1.00 MPa of the gas phase in the head space of the biogas reactor,
wherein the feedstock and/or the feedstock slurry has a C/N ratio of at most 15 wherein C is the total carbon and is determined according to DIN ISO 10694 and N is the total nitrogen and is determined according to DIN ISO 13878, and in that the feedstock is an organic waste mixture containing at least 60 wt% of poultry manure based on the total weight of the mixture.

The inventive process can be conducted in a plant 1 comprising optional means to provide feedstock 3 and an aqueous solution 5 to a collection tank 7 provided with agitating means 9 in order to form a feedstock slurry 11. The feedstock 3 is indeed diluted with the purpose of being pumpable, not in order to lower the ammonium concentration. Thus, when the feedstock 3 is pumpable as such, the dilution to obtain a feedstock slurry 11 may not be necessary. The plant 1 further comprises means 19 to supply the biogas reactor 13 with feedstock slurry 11 or feedstock 3 (i.e. a feedstock or feedstock slurry feeder 19) configured to supply said feedstock 3 or feedstock slurry 11 at least in an amount via which content of ammonium of at least 2.7 g/l as determined according to DIN 38406-5 is set in the liquid phase 15 of the biogas reactor 13. Said a feedstock or feedstock slurry feeder 19 comprises dosing means 21 to mix one or more organic waste in selected amounts in order to form the feedstock and/or to dilute said feedstock 3 with an appropriate amount of water 5 thereby forming the feedstock slurry 11 and optionally means 23 to analyze the ammonium content of the feedstock slurry 11, and a pump (not represented) to introduce the feedstock 3 or feedstock slurry 11 to at least one pressurizable biogas reactor 13. The dosing means can be controlled by a computer 25. In a preferred embodiment the plant 1 comprises only one biogas reactor 13.

A pressurizer 27 is associated to each biogas reactor 13 and means 29 to monitor the pressure of the gas phase 17 in said associated biogas reactor 13 configured to act on the pressurizer 27 in order to keep a pressure of at least 0.13 MPa, preferably at least 0.25 MPa, of the gas phase 17 in the head space of the biogas reactor 13.

Preferably, the pressurizer is the feedstock or feedstock slurry feeder or an external compressed gas source or a fluid inlet or any combination thereof. In a preferred embodiment the pressurizer is the feedstock or feedstock slurry feeder.

As used herein the term "feedstock" is taken to mean any organic waste material such as animal or plant material or mixture of animal and/or plant material that contains one or more components that can be converted into biogas through an anaerobic fermentation process in a biogas reactor. The feedstock can comprise one or more selected from animal tissue, biomass, fish tissue or part, plant parts, fruits, vegetables, plant processing wastes, animal processing wastes, animal manure or urine, mammalian manure or urine, solids isolated from fermentations cultures and combination thereof.

Particular examples of feedstock according to the invention include bovine, poultry, equine and porcine manure or urine, wood saving or chips, slops, mostos, shredded paper, cotton burrs, grain, chaff, seed shells, hay, alfalfa, grass, leaves, sea shells, seed pods, corn shucks, weeds, aquatic plants, algae, fungus and combination thereof. Preferably, the feedstock according to the invention is animal manure such as poultry or chicken manure, or combination of animal manure with wood saving or chips, slops, mostos, shredded paper, cotton burrs, grain, chaff, seed shells, hay, alfalfa, grass, leaves, sea shells, seed pods, corn shucks, weeds, aquatic plants, algae, fungus, wherein the animal manure or the resulting mixture has a C/N ratio of at most 15, preferably of at most 12, and more preferably of at most 10.

According to the invention the feedstock is an organic waste mixture containing at least 60 wt% of poultry manure based on the total weight of the mixture, preferably at least 70 wt% of poultry manure, more preferably at least 80 wt% of poultry manure. More preferably, poultry manure is chicken manure.

A feedstock slurry is prepared by suspending a feedstock in an aqueous solution to form a slurry containing less than 85 wt% of solids, preferably from 0.1 to 50 wt% of solids, more preferably from 1 to 35 wt% of solids based on the total weight of the feedstock slurry. In accordance with the invention the aqueous solution is preferably water such as fresh or recycled water.

The particle size of the feedstock can optionally be reduced either prior or during the preparation of the feedstock slurry by any means known to the person skilled in the art such as a grinder for example. No particular size is required for the feedstock. However, smaller particle sizes are preferred as smaller particles are generally digested more quickly than larger particles. Therefore, in an embodiment of the invention, the particle size of the feedstock is less than 3 mm.

Anaerobic bacteria are bacteria capable of transforming the feedstock into biogas, such as for example methane, into an anaerobic biogas reactor. According to the invention the anaerobic bacteria can be either mesophile or thermophile, preferably the anaerobic bacteria is thermophile.

Example of anaerobic bacteria that can be used in a biogas reactor or anaerobic biogas reactor include yeast, a methanogenic bacterium, methanobacterium acetobacterium, acetogenic bacterium, liquefaction bacterium, Clostridium spp. (methane), Bacillus spp., Escherichia spp., Staphylococcus spp., Methanobacter spp., Methanobacter (Mb.) omlianskii (methane), Mb. formicicum (methane), Mb. soehngenii (methane), Mb. thermoautrophicum (methane), Mb. ruminatium (methane), Mb. mobile (methane), Mb. methanica (methane), Methanococcus (Mc.) mazei (methane), Mc. vannielii (methane), Ms. mazei (methane), Mb. suboxydans (methane), Mb. propionicum (methane), Methanosarcina (Ms.) bovekeri (methane), Ms. methanica (methane), Ms. alcaliphilum (methane), Ms. acetivorans (methane), Ms. thermophilia (methane), Ms. barkeri (methane), Ms. vacuolata (methane), Propionibacterium acidi-propionici (methane), Saccharomyces cerevisae (ethanol), S. ellipsoideus (ethanol), Clostridium propionicum (propanol), Clostridium saccharoacetoper-butylicum (butanol), Clostridium butyricum (hydrogen), wherein the chemical in parentheses indicates a useful material which that bacteria produces.

In a preferred embodiment of the invention, the feedstock is animal manure, preferably poultry manure, or the feedsock is an organic waste mixture containing at least 60 wt% of poultry manure based on the total weight of the mixture; and no anaerobic bacteria is added to the biogas reactor. Fermentation is conducted in the biogas reactor without inoculation of the biogas reactor with additional anaerobic bacteria. Fermentation is conducted with the original bacteria content of the feedstock.

In another embodiment, the biogas reactor is inoculated with an anaerobic bacteria, with preference with thermophile anaerobic bacteria,
The anaerobic fermentation or digestion takes place in a biogas reactor. The classic biogas reactor contains about 1,000 to 5,000 m³. Fermentation is conducted under mesophilic or thermophilic conditions. Thus the process is conducted at temperatures ranging from ambient temperature (i.e. about 20°C) to 80°C. Preferably, the process is performed under thermophilic conditions, at temperatures ranging from 45 to 80°C, preferably from 50 to 70°C more preferably from 53 to 60°C. For example, the inventive process can conducted at the temperature of 55°C.

In accordance with the invention, the fermentation process is conducted at a pressure of at least 0.13 MPa, preferably at least 0.20 MPa, more preferably 0.25 MPa, even more preferably of at least 0.28 MPa, even more preferably of at least 0.30 MPa, and most preferably of at least 0.32 MPa. The fermentation process is conducted at a pressure of at most 1.00 MPa, preferably of at most 0.80 MPa, more preferably of at most 0.60 MPa. For an optimal methane production, the person skilled in the art would advantageously conduct the fermentation process at high pressure, such as at a pressure of at least 0.25 MPa. In a preferred embodiment, the anaerobic fermentation is conducted at a pH ranging from 6 to 8.5, preferably at a pH ranging from 7 to 8. Without being bound by a theory, it is believed that the HCO₃ buffer formed by the increased CO₂ solution, at elevated pressure, effectively stabilizes the pH within the described ranges. Thus according to a preferred embodiment of the invention, no chemicals are added in the biogas reactor in order to stabilize the pH within the range of from 6 to 8.5, preferably of from 7 to 8.

Biogas produced by the fermentation is removed from the biogas reactor 13 via an outlet 31 at the top of the reactor vessel. The collecting of the biogas is done according to conventional methods known from the person skilled in the art and therefore not described any further in the present description.

The biogas reactor 13 is provided with agitating means 33 and recirculation pumps (not represented) in order to have the reaction solution constantly in movement. This is to avoid the sedimentation of the solids contained in the reaction solution and the genesis of a film or crust on the liquid surface that could disturb the collection of the produced biogas.

In accordance with the invention the amount of solids in the biogas reactor 13 is ranging from 1 to 70 wt%, preferably from 10 to 60 wt% based upon the total weight of the solution.

The biogas reactor 13 is supplied with feedstock 3 or feedstock slurry 11 at least in an amount via which content of ammonium of at least 2.7 g/l as determined according to DIN 38406-5 is set in the liquid phase 15 thus the biogas reactor is optionally provided with means 35 to control the ammonium content of the liquid phase 15. These means 35 are configured to act on the dosing means 21 via the computer 25. Such a content of ammonium of at least 2.7 g/l in the liquid phase 15 of the biogas reactor 13 was known in prior art to result in fermentation inhibition. Surprisingly, the inventors have discovered that when the fermentation is conducted at a pressure of at least 0.13 MPa in the gas phase 17, fermentation inhibition was avoided or at least reduced. Preferably, the biogas reactor 13 is supplied with feedstock 3 or feedstock slurry 11 at least in an amount via which content of ammonium of at least 3.0 g/l is set in the liquid phase, preferably of at least 3.5 g/l even more preferably of at least 4.0 g/l. The person skilled in the art would have advantage to raise the pressure to at least 0.25 MPa in order to enhance fermentation in the biogas reactor.

The residence time in the biogas reactor 13 will vary depending on several factors, such as the nature of the feedstock. For example the average residence time is about 10 to 40 days. The reduction of the average residence time coupled to a low C/N ratio of the feedstock and/or the feedstock slurry used in accordance to the invention allows a reduction of the classic biogas reactor size.

In accordance with the invention the biogas reactor 13 is provided with a steady flow of feedstock 3 or feedstock slurry 11. In order to have a continuous process, the biogas reactor is supplied with a continuous flow of feedstock slurry, and the same volume of liquid is removed continuously as digestate 37. The volume of the biogas reactor and the rate flow entering (feed rate) or exiting (effluent rate) the biogas reactor are calculated to have the average desired residence time in the biogas reactor for proceeding with the fermentation.

It is understood from the above that the process according to the invention is preferably conducted in a continuous mode, i.e. wherein the effluent rate will not exceed the feed rate.

However, it can also be conducted in a semi continuous mode wherein feedstock is added to the biogas reactor incrementally and gas and/or digestate incrementally at the same or different times. The process of the invention can also be conducted in batch mode wherein larger portions of feedstock are added to the reactor at given time intervals and larger portions of gas and digestate are removed from the reactor at the same or different time intervals. During continuous operation, the operating temperature and rate of gas production will be relatively constant. Generally continuous operation will provide greater rate of gas production than batch or semi-continuous operation.

Once the anaerobic fermentation is done, the digestate 37 contained in the biogas reactor 13 is pumped, filtered by filtration means 39 before being treated by ultrafiltration 41 and reverse osmosis 43 in order to recover water 45, in accordance to methods known to the person skilled in the art. An example of treatment of the digestate suitable according to the invention is described in EP2390235 which is incorporated herein by reference.

### Test methods

The content of ammonium in the liquid phase of the biogas reactor is measured according to DIN 38406-5.

The C/N ratio of the feedstock and/or the feedstock slurry is determined according to the following: C is the total carbon and is determined according to DIN ISO 10694 and N is the total nitrogen and is determined according to DIN ISO 13878.

### Example

### Anaerobic digestion of chicken manure

The process was conducted in a biogas fermenter. 350 kg of chicken manure was diluted in 350 I of fresh water and then loaded in a biogas reactor equipped with an internal mechanical agitator and a heat controller. The C/N ratio of the chicken manure used was determined to be 8.5. The percent solid of the reaction solution was 18 wt% based of the solution weight.

The biogas reactor was run for a period of 1300 hours with periodic sampling of the gas phase in the head space and of the liquid phase. The temperature was maintained at a constant temperature of 55°C. Analysis of the liquid phase at t = 1200 hours showed an ammonium content of 5.158 g/l. The pH of the liquid phase was 7.97.

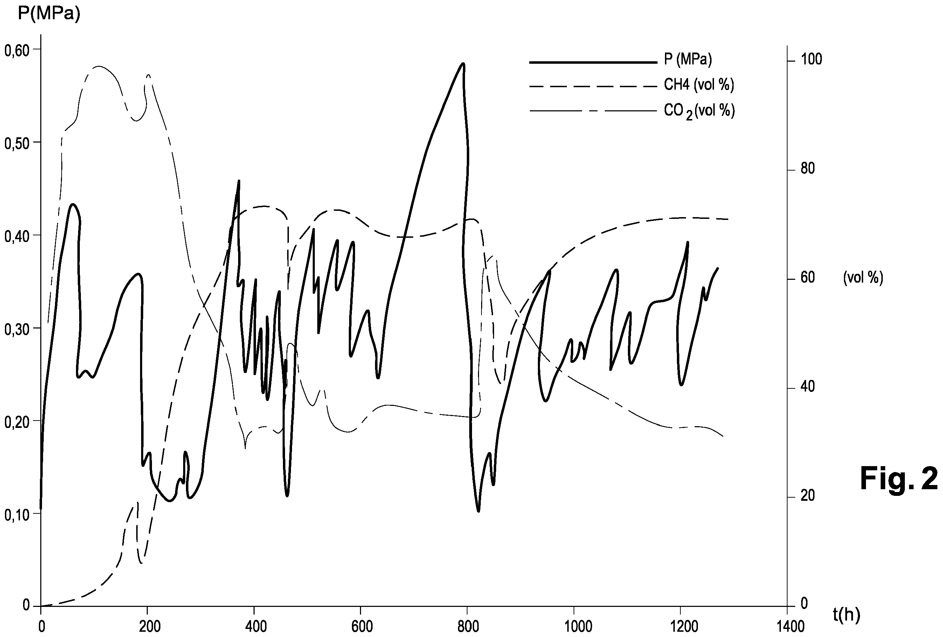

Pressure of the gas phase was kept over 0.25 MPa and for most of the time between 0.26 and 0.35 MPa. Two drops of pressure to 0.1 MPa were performed at t = 420 hours and at t = 850 hours in order to better determine the influence of pressure in the CH₄ production. The results of the experiment is given in Fig. 2. From figure 2 it can be easily seen that the CH₄ content decreases whereas the CO₂ content in the produced gas increases during the drop of pressure.

The total amount of methane produced was about 4.5 m³ after about 1200 hours.

## Claims

1. Process for treating a feedstock (3) in a liquid form comprising the steps of:
- providing a feedstock (3);
- optionally diluting the feedstock (3) by addition of an aqueous solution (5) in order to obtain a feedstock slurry (11), and
- subjecting the feedstock (3) or the feedstock slurry (11) to an anaerobic fermentation in a biogas reactor (13) thereby forming a liquid phase (15) and gas phase (17),
the process being **characterized in that** the biogas reactor (13) is supplied with feedstock (3) or feedstock slurry (11) at least in an amount via which content of ammonium of at least 2.7 g/l as determined according to DIN 38406-5 is set in the liquid phase (15) and **in that** the anaerobic fermentation is conducted at a pressure of at least 0.13 MPa and of at most 1.00 MPa of the gas phase (17) in the head space of the biogas reactor (13), **in that** the feedstock (3) and/or the feedstock slurry (11) has a C/N ratio of at most 15 wherein C is the total carbon and is determined according to DIN ISO 10694 and N is the total nitrogen and is determined according to DIN ISO 13878, and **in that** the feedstock (3) is an organic waste mixture containing at least 60 wt% of poultry manure based on the total weight of the mixture.

2. Process according to claim 1 **characterized in that** the feedstock (3) and/or the feedstock slurry (11) has a C/N ratio of at most 12, preferably of at most 10, wherein C is the total carbon and is determined according to DIN ISO 10694 and N is the total nitrogen and is determined according to DIN ISO 13878.

3. Process according to claim 1 or 2 **characterized in that** the feedstock (3) is animal manure, preferably poultry manure, more preferably chicken manure.

4. Process according to claim 1 or 2 **characterized in that** the feedstock (3) is an organic waste mixture containing at least 60 wt% of chicken manure, based on the total weight of the mixture.

5. Process according to any one of claims 3 or 4, **characterized in that** the anaerobic fermentation is conducted with the anaerobic bacteria content of the feedstock and that no additional anaerobic bacteria is inoculated in the biogas reactor.

6. Process according to any one of claims 1 to 5 **characterized in that** the process is a thermophilic process conducted at a temperature ranging from 45 to 80°C.

7. Process according to any one of claims 1 to 6 **characterized in that** the anaerobic fermentation is conducted at a pH ranging from 6 to 8.5

8. Process according to any one of claims 1 to 7 **characterized in that** the pressure of the gas phase (17) in the head space of the biogas reactor (13) is of at least 0.25 MPa, preferably at least 0.30 MPa, more preferably at least 0.32 MPa.

9. Process according to any one of claims 1 to 8 **characterized in that** the biogas reactor (13) is supplied with feedstock (3) or feedstock slurry (11) at least in an amount via which content of ammonium of at least 3.0 g/l is set in the liquid phase, preferably of at least 3.5 g/l.

10. Process according to any one of claims 1 to 9 **characterized in that** the anaerobic fermentation is a one phase anaerobic digestion.

## Patentansprüche

1. Verfahren zur Behandlung eines Rohstoffs (3) in flüssiger Form, umfassend die Schritte:
- Bereitstellen eines Rohstoffs (3);
- gegebenenfalls Verdünnen des Rohstoffs (3) durch Zugabe einer wässrigen Lösung (5), um eine Rohstoffaufschlämmung (11) zu erhalten, und
- Unterwerfen des Rohstoffs (3) oder der Rohstoffaufschlämmung (11) einer anaeroben Fermentation in einem Biogasreaktor (13), wodurch eine flüssige Phase (15) und eine Gasphase (17) gebildet werden,
wobei das Verfahren **dadurch gekennzeichnet ist, dass** dem Biogasreaktor (13) Rohstoff (3) oder Rohstoffaufschlämmung (11) mindestens in einer Menge zugeführt wird, bei der ein nach DIN 38406-5 bestimmten Mindestgehalt an Ammonium von 2,7 g/l in der flüssigen Phase (15) festgestellt wird, und dadurch dass die anaerobe Fermentation bei einem Druck von mindestens 0,13 MPa und höchstens 1,00 MPa in der Gasphase (17) im Kopfraum des Biogasreaktors (13) durchgeführt wird, dadurch dass der Rohstoff (3) und/oder die Rohstoffaufschlämmung (11) ein C/N-Verhältnis von höchstens 15 aufweist, wobei C der Gesamtkohlenstoff ist und gemäß DIN ISO 10694 bestimmt wird und N der Gesamtstickstoff ist und gemäß DIN ISO 13878 bestimmt wird, und dass der Rohstoff (3) ein organisches Abfallgemisch ist, das mindestens 60 Gew.-% Geflügelmist, bezogen auf das Gesamtgewicht des Gemisches, enthält.

2. Verfahren nach Anspruch 1, **dadurch gekennzeichnet, dass** der Rohstoff (3) und/oder die Rohstoffaufschlämmung (11) ein C/N-Verhältnis von höchstens 12, vorzugsweise von höchstens 10 aufweisen, wobei C der Gesamtkohlenstoff ist und gemäß DIN ISO 10694 bestimmt wird und N ist der Gesamtstickstoff und nach DIN ISO 13878 bestimmt wird.

3. Verfahren nach Anspruch 1 oder 2, **dadurch gekennzeichnet, dass** der Rohstoff (3) Tiermist ist, vorzugsweise Geflügelmist, besonders bevorzugt Hühnermist.

4. Verfahren nach Anspruch 1 oder 2, **dadurch gekennzeichnet, dass** der Rohstoff (3) ein organisches Abfallgemisch ist, das mindestens 60 Gew.-% Hühnermist bezogen auf das Gesamtgewicht des Gemisches enthält.

5. Verfahren nach einem der Ansprüche 3 oder 4, **dadurch gekennzeichnet, dass** die anaerobe Fermentation mit dem anaeroben Bakteriengehalt des Rohstoffs durchgeführt wird und dass im Biogasreaktor keine zusätzlichen anaeroben Bakterien inokuliert werden.

6. Verfahren nach einem der Ansprüche 1 bis 5, **dadurch gekennzeichnet, dass** das Verfahren ein thermophiles Verfahren ist, das bei einer Temperatur im Bereich von 45 bis 80 ° C durchgeführt wird.

7. Verfahren nach einem der Ansprüche 1 bis 6, **dadurch gekennzeichnet, dass** die anaerobe Fermentation bei einem pH-Wert im Bereich von 6 bis 8,5 durchgeführt wird

8. Verfahren nach einem der Ansprüche 1 bis 7, **dadurch gekennzeichnet, dass** der Druck der Gasphase (17) im Kopfraum des Biogasreaktors (13) mindestens 0,25 MPa beträgt, vorzugsweise mindestens 0,30 MPa, bevorzugter mindestens 0,32 MPa.

9. Verfahren nach einem der Ansprüche 1 bis 8, **dadurch gekennzeichnet, dass** dem Biogasreaktor
(13) Rohstoff (3) oder Rohstoffaufschlämmung (11) mindestens in einer Menge zugeführt wird, bei der ein Ammoniumgehalt von mindestens 3,0 g/l in der flüssigen Phase festgestellt wird, vorzugsweise von mindestens 3,5 g/l.

10. Verfahren nach einem der Ansprüche 1 bis 9, **dadurch gekennzeichnet, dass** die anaerobe Fermentation ein einphasiger anaerober Abbau ist.

## Revendications

1. Procédé de traitement d'une charge d'alimentation (3) sous forme liquide comprenant les étapes consistant à :
- prévoir une charge d'alimentation (3) ;
- optionnellement diluer la charge d'alimentation (3) par addition d'une solution aqueuse (5) afin d'obtenir une suspension de charge d'alimentation (11), et
- soumettre la charge d'alimentation (3) ou la suspension de charge d'alimentation (11) à une fermentation anaérobie dans un réacteur de biogaz (13) formant ainsi une phase liquide (15) et une phase gazeuse (17),
le procédé étant **caractérisé en ce que** le réacteur de biogaz (13) est alimenté avec la charge d'alimentation (3) ou avec la suspension de charge d'alimentation (11) au moins en une quantité par l'intermédiaire de laquelle une teneur en ammonium d'au moins 2,7 g/l, déterminée selon la norme DIN 38406-5, soit définie dans la phase liquide (15) et **en ce que** la fermentation anaérobie est mise en oeuvre à une pression d'au moins 0,13 MPa et d'au plus 1,00 MPa de la phase gazeuse (17) dans l'espace de tête du réacteur de biogaz (13), **en ce que** la charge d'alimentation (3) et/ou la suspension de charge d'alimentation (11) a un rapport C/N valant au plus 15, dans lequel C est le carbone total et étant déterminé selon la norme DIN ISO 10694 et N est l'azote total et étant déterminé selon la norme DIN ISO 13878, et **en ce que** la charge d'alimentation (3) est un mélange de déchets organiques contenant au moins 60% en poids de lisier de volaille, sur la base du poids total du mélange.

2. Procédé selon la revendication 1, **caractérisé en ce que** la charge d'alimentation (3) et/ou la suspension de charge d'alimentation (11) a un rapport C/N valant au plus 12, de préférence valant au plus 10, dans lequel C est le carbone total et étant déterminé selon la norme DIN ISO 10694 et N est l'azote total et étant déterminé selon la norme DIN ISO 13878.

3. Procédé selon la revendication 1 ou 2, **caractérisé en ce que** la charge d'alimentation (3) est du lisier animal, de préférence du lisier de volaille, de façon plus préférée du lisier de poulet.

4. Procédé selon la revendication 1 ou 2, **caractérisé en ce que** la charge d'alimentation (3) est un mélange de déchets organiques contenant au moins 60% en poids de lisier de poulet, sur la base du poids total du mélange.

5. Procédé selon l'une des revendications 3 et 4, **caractérisé en ce que** la fermentation anaérobie est mise en oeuvre avec la teneur en bactéries anaérobies de la charge d'alimentation et qu'aucune bactérie anaérobie supplémentaire n'est inoculée dans le réacteur de biogaz.

6. Procédé selon l'une quelconque des revendications 1 à 5, **caractérisé en ce que** le procédé est un procédé thermophile mis en oeuvre à une température située dans la plage allant de 45 à 80° C.

7. Procédé selon l'une quelconque des revendications 1 à 6, **caractérisé en ce que** la fermentation anaérobie est mise en oeuvre à un pH situé dans la plage allant de 6 à 8.5.

8. Procédé selon l'une quelconque des revendications 1 à 7, **caractérisé en ce que** la pression de la phase gazeuse (17) dans l'espace de tête du réacteur de biogaz (13) est d'au moins 0,25 MPa, de préférence d'au moins 0,30 MPa, de façon plus préférée d'au moins 0,32 MPa.

9. Procédé selon l'une quelconque des revendications 1 à 8, **caractérisé en ce que** le réacteur de biogaz (13) est alimenté en la charge d'alimentation (3) ou en la suspension de charge d'alimentation (11) au moins en une quantité par l'intermédiaire de laquelle une teneur en ammonium d'au moins 3,0 g/l soit définie dans la phase liquide, de préférence d'au moins 3,5 g/l.

10. Procédé selon l'une quelconque des revendications 1 à 9, **caractérisé en ce que** la fermentation anaérobie est une digestion anaérobie monophase.
